# EUROPEAN PATENT SPECIFICATION

(11) **EP 3 257 550 B1**
(45) Date of publication and mention of the grant of the patent: **21.11.2018**
(21) Application number: 16174766.2
(22) Date of filing: 16.06.2016
(51) Int. Cl.: A61N 1/04, A61N 1/32, A61N 1/36

(54) **AN APPARATUS CONFIGURED TO ENABLE A FLOW OF CURRENT THROUGH A USER'S SKIN**
VORRICHTUNG ZUR ERMÖGLICHUNG EINES STROMFLUSSES DURCH DIE HAUT EINES BENUTZERS
APPAREIL CONFIGURÉ POUR PERMETTRE UN FLUX DE COURANT À TRAVERS LA PEAU D'UN UTILISATEUR

(43) Date of publication of application: 20.12.2017
(73) Proprietor: Nokia Technologies Oy, 02610 Espoo (FI)
(72) Inventor: Radivojevic, Zoran, Cambridge CB3 0NP (GB)
(74) Representative: Potter Clarkson LLP

(56) References cited:
- EP-A1- 3 016 179
- WO-A2-2009/026588
- US-A1- 2013 013 022
- US-A1- 2014 349 211

## Description

### Technical Field

The present disclosure relates to the use of proton batteries and associated apparatus, and in particular concerns an apparatus comprising a proton battery and a first pair of electrical contacts configured to enable a flow of current through a user's skin when the apparatus is attached to the skin of the user.

### Background

Electrical stimulation can be provided/applied to a person's skin for various purposes, including therapeutic purposes. One example of such electrical stimulation is transcutaneous electrical nerve stimulation (TENS). US2013/013022 A1 shows an electrostimulation device.

The listing or discussion of a prior-published document or any background in this specification should not necessarily be taken as an acknowledgement that the document or background is part of the state of the art or is common general knowledge.

### Summary

According to a first aspect, there is provided an apparatus comprising:
a proton battery configured to generate protons in the presence of water to produce a potential difference; and
a first pair of electrical contacts electrically connected to the proton battery to enable a flow of electrical current therebetween when the potential difference from the proton battery is applied across the first pair of electrical contacts,
wherein the apparatus is configured such that when the apparatus is attached to the skin of a user, the proton battery is exposed to water from the user's skin which produces the potential difference and enables a flow of electrical current through the user's skin via the first pair of electrical contacts.

The proton battery may be a graphene thin film battery.

The proton battery may comprise a first electrode, a second electrode and an electrolyte,
the first electrode comprising graphene oxide and configured to generate protons in the presence of water to produce the potential difference between the first and second electrodes, and
the electrolyte configured to enable the generated protons to flow from the first electrode to the second electrode when the first and second electrodes are connected by an external circuit.

The second electrode may comprise reduced graphene oxide.

The electrolyte may be configured to absorb water from the surrounding environment and deliver it to the first electrode to facilitate the generation of protons. The electrolyte may comprise one or more of a room-temperature ionic fluid and an ion gel.

The apparatus may further comprise a porous substrate, wherein the apparatus is configured such that when the apparatus is attached to the skin of a user the proton battery is exposed to water from the user's skin via the porous substrate.

The porous substrate may comprise one or more of a textile-like material, a semi-permeable woven material, a Gore-Tex™ membrane and a semi-permeable polymeric membrane.

The apparatus may further comprise a controller element which is electrically connected between the proton battery and the first pair of electrical contacts and is configured to control the potential difference applied across the first pair of electrical contacts by the proton battery and the resulting flow of electrical current through the user's skin.

The controller element may comprise electrical stimulation driving electronics. The electrical stimulation driving electronics may control the flow of electrical current through the user's skin according to a predetermined pattern. The driving electronics may comprise one or more of an RC oscillator, an inductive solenoid, a transformer, a current-limiting circuit and miniature DC-DC voltage booster circuitry.

The apparatus may be configured to enable a flow of electrical current through the user's skin at a level which causes one or more of:
stimulation of nerves in the user's skin;
stimulation of muscles proximal to the user's skin;
alteration of properties of the user's skin; and
alteration of properties of blood vessels proximal to the user's skin.

The electrical stimulation driving electronics may be transcutaneous electrical nerve stimulation driving electronics. The apparatus may be configured to enable a flow of electrical current through the user's skin at a level which is consistent with transcutaneous electrical nerve stimulation therapy.

The controller element is one of detachable from the apparatus and not detachable from the apparatus.

The controller element may be detachable from the apparatus via respective magnetic contacts within the controller element and the apparatus.

The apparatus may further comprise one or more additional pairs of electrical contacts electrically connected to the proton battery to enable a flow of electrical current through the user's skin via the respective one or more pairs of electrical contacts when the potential difference from the battery is applied across the respective one or more additional pairs of electrical contacts.

The apparatus may further comprise attachment means for temporarily attaching the apparatus to the user's skin.

The apparatus may further comprise a medicament configured to be absorbed into the user's skin. The apparatus may be configured such that the flow of electrical current through the user's skin improves the absorption of the medicament into the user's skin.

The apparatus may further comprise at least one removable protective layer configured to prevent the proton battery from being undesirably exposed to water (e.g. from the air) when not attached to a user's skin. The apparatus may comprise a first protective layer configured to cover the electrical contacts. The apparatus may comprise a second protective layer configured to cover one or more of the proton battery and the controller element.

The apparatus may comprise one or more of a patch, a plaster, a sticker, a bandage, an elastic band, clothing and socks.

The proton battery may be configured to produce a potential difference in the range of 0.1-10 V.

The apparatus may be configured to have an effective lifetime of between 10 and 60 minutes.

The apparatus may be configured to be disposable. The apparatus may be configured to be resealable and reusable.

According to a further aspect, there is provided a method of making an apparatus, the method comprising:
electrically connecting a first pair of electrical contacts to a proton battery to enable a flow of electrical current therebetween when a potential difference from the proton battery is applied across the first pair of electrical contacts, the proton battery configured to generate protons in the presence of water to produce the potential difference; and
configuring the apparatus such that when the apparatus is attached to the skin of a user, the proton battery is exposed to water from the user's skin which produces the potential difference and enables a flow of electrical current through the user's skin via the first pair of electrical contacts.

According to a further aspect, there is provided a method of using an apparatus,
the apparatus comprising:
   a proton battery configured to generate protons in the presence of water to produce a potential difference; and
   a first pair of electrical contacts electrically connected to the proton battery to enable a flow of electrical current therebetween when the potential difference from the proton battery is applied across the first pair of electrical contacts,
   wherein the apparatus is configured such that when the apparatus is attached to the skin of a user, the proton battery is exposed to water from the user's skin which produces the potential difference and enables a flow of electrical current through the user's skin via the first pair of electrical contacts,
the method comprising attaching the apparatus to the skin of a user such that the proton battery is exposed to water from the user's skin to produce a potential difference and enable a flow of electrical current through the user's skin via the first pair of electrical contacts.

According to a further aspect, there is provided a computer program configured to control the operation of an apparatus as described above to enable a flow of electrical current through a user's skin and/or to provide electrical stimulation to a user's skin.

The steps of any method disclosed herein do not have to be performed in the exact order disclosed, unless explicitly stated or understood by the skilled person.

Corresponding computer programs for implementing one or more steps of the methods disclosed herein are also within the present disclosure and are encompassed by one or more of the described example embodiments.

One or more of the computer programs may, when run on a computer, cause the computer to configure any apparatus, including a battery, circuit, controller, or device disclosed herein or perform any method disclosed herein. One or more of the computer programs may be software implementations, and the computer may be considered as any appropriate hardware, including a digital signal processor, a microcontroller, and an implementation in read only memory (ROM), erasable programmable read only memory (EPROM) or electronically erasable programmable read only memory (EEPROM), as non-limiting examples. The software may be a manufacture or assembly program.

One or more of the computer programs may be provided on a computer readable medium, which may be a physical computer readable medium such as a disc or a memory device, or may be embodied as a transient signal. Such a transient signal may be a network download, including an internet download.

The present disclosure includes one or more corresponding aspects, example embodiments or features in isolation or in various combinations whether or not specifically stated (including claimed) in that combination or in isolation. Corresponding means for performing one or more of the discussed functions are also within the present disclosure.

The above summary is intended to be merely exemplary and non-limiting.

### Brief Description of the Figures

A description is now given, by way of example only, with reference to the accompanying drawings, in which:-
Figure 1a shows a photograph of a traditional TENS machine including wired electrodes;
Figure 1b shows a photograph of a TENS machine with a remote controller;
Figure 2 illustrates schematically one example of the present apparatus;
Figure 3 illustrates schematically one example of the pair of electrical contacts in plan view;
Figure 4 illustrates schematically another example of the present apparatus;
Figure 5 illustrates schematically another example of the present apparatus;
Figure 6 illustrates schematically another example of the present apparatus;
Figure 7 illustrates schematically another example of the present apparatus;
Figure 8 illustrates schematically one example of a proton battery in the present apparatus;
Figure 9 shows the main steps of a method of making the present apparatus;
Figure 10 shows the main steps of a method of using the present apparatus; and
Figure 11 shows a computer-readable medium comprising a computer program configured to perform, control or enable a method described herein.

### Description of Specific Aspects/Embodiments

Electrical stimulation can be provided/applied to a person's skin for various purposes, including for therapeutic purposes. One example of such electrical stimulation is transcutaneous electrical nerve stimulation (TENS). Another example is neuromuscular electrical stimulation (NMES) (also known as electrical muscle stimulation - EMS).

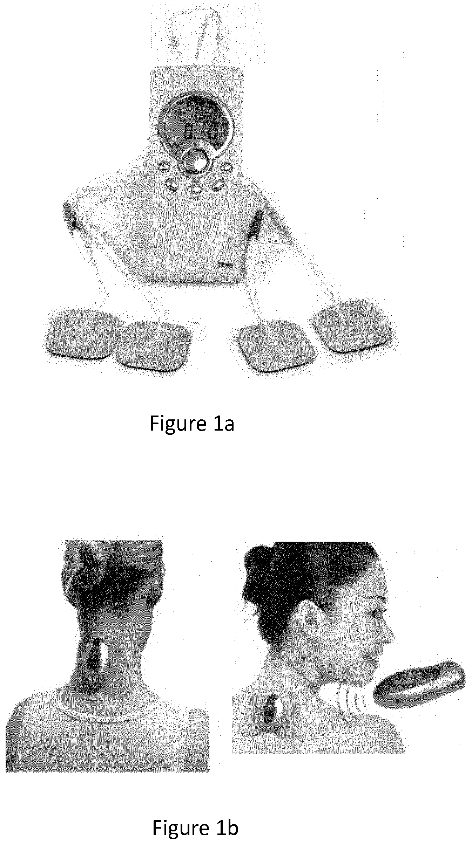

Transcutaneous electrical nerve stimulation involves applying electrical current to a person's skin in order to stimulate nerves proximal to the skin. This may be done for therapeutic purposes including, most commonly, to alleviate pain. A traditional TENS machine, such as the machine shown in Figure 1a, includes a battery-operated (e.g. AA batteries) unit which is connected to the skin using two or more electrodes. The electrodes are connected to the TENS unit by wires.

However, traditional wired TENS machines can be difficult, cumbersome and uncomfortable for a person to use, particularly without assistance from another person. For example, it may be difficult for a user to view and control the TENS unit when the wired electrodes are placed on the user's back. Additionally, it may be difficult, cumbersome and embarrassing for a user to use a wired TENS machine in public, and so such TENS machines are essentially limited to non-public use. Further, traditional TENS machines involve many parts (TENS unit, multiple sets of electrodes, multiple sets of wires) which makes the machine more complicated and complex for a user to use.

TENS machines which use a remote controller to control the TENS stimulation have been developed, such as the system shown in Figure 1b. However, a remote controller system still provide several problems. For example, the remote controller is required to control the TENS machine and so if the remote controller is lost, damaged or runs out of power, the TENS machine cannot be used/operated.

Neuromuscular electrical stimulation involves applying electrical current/impulses to a person's skin in order to cause muscle contraction. This may be done for various purposes (e.g. for training, therapeutic or cosmetic reasons) including as a rehabilitation tool for immobilised patients and as a post-exercise recovery tool for athletes. Traditional NMES machines are similar to traditional TENS machines involving a NMES unit and multiple wired electrodes, and so NMES machines provide similar problems.

There will now be described an apparatus that may provide a solution to these problems or to other problems.

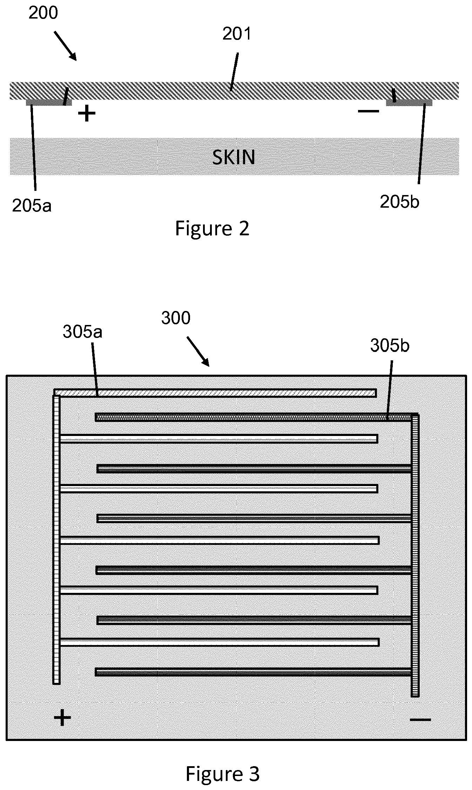

Figure 2 shows one example of the present apparatus 200, including a proton battery 201 and a pair of electrical contacts 205a and 205b which are electrically connected (not shown) to the proton battery 201. The apparatus may be attached to, applied to or positioned on a user's skin such that the electrical contacts are in (direct) contact with the user's skin.

The proton battery 201 is configured to generate protons in the presence of water to produce a potential difference and the first pair of electrical contacts 205a, 205b are configured enable a flow of electrical current therebetween when the potential difference from the proton battery 201 is applied across the first pair of electrical contacts and when the electrical contacts are connected by an external circuit. The apparatus 200 is configured such that when the apparatus 200 is attached to the skin of a user, the proton battery 201 is exposed to water from the user's skin which produces the potential difference and enables a flow of electrical current through the user's skin via the first pair of electrical contacts 205a, 205b. That is, the proton battery is automatically "activated" (i.e. automatically begins to generate protons and produce a potential difference) by water/moisture from the user's skin.

Figure 3 shows, from a plan view, one example configuration 300 of the electrical contacts 305a and 305b. In this example the two electrical contacts are interdigitated to increase the area of electrical contact with the skin when the apparatus is applied to a user's skin. Of course, other electrical contacts configurations are possible and within the scope of this disclosure.

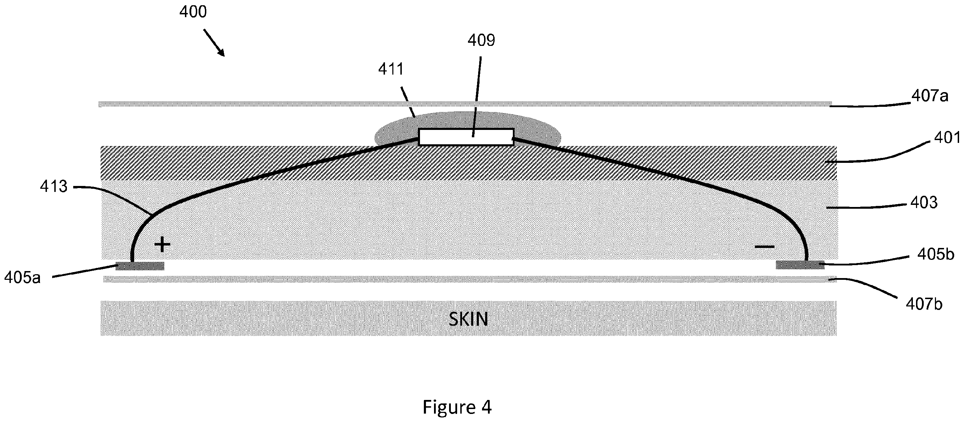

Figure 4 shows another example of the present apparatus 400 including a proton battery 401 and a first pair of electrical contacts 405a and 405b which are electrically connected (not shown) to the proton battery 401. The apparatus also includes a porous substrate 403, upper protective layer 407a, lower protective layer 407b, controller element 409, glob top encapsulation 411 and connection wires 413.

The proton battery 401 is connected to (not shown) and provides power to the controller element 409. The controller element 409 receives power from the proton battery 401 and provides power to the first pair of electrical contacts 405a, 405b (to which it is connected by connection wires 413). More specifically, the controller element 409 is configured to control the potential difference applied across the first pair of electrical contacts 405a, 405b by the proton battery 401 and the resulting flow of electrical current through the user's skin.

The controller element 409 may comprise electrical stimulation driving electronics which control or regulate the flow of electrical current through the user's skin according to a predetermined pattern. The driving electronics may comprise one or more of an RC oscillator, an inductive solenoid, a transformer, a voltage booster and a current limiting circuit. The electrical stimulation driving electronics may control the pattern, intensity/amplitude or frequency of the current. In one mode of operation (a 'pulsed' mode), the driving electronics may deliver multiple signal bursts where each pulse is formed as multiple pulse trains of very short pulses (1-1000 microseconds each). In another mode of operation ('DC' mode), the driving electronics may deliver long-lasting unipolar signals. DC mode may be more suitable than pulsed mode when the apparatus is used to provide accelerated medicament adsorption into the user's skin.

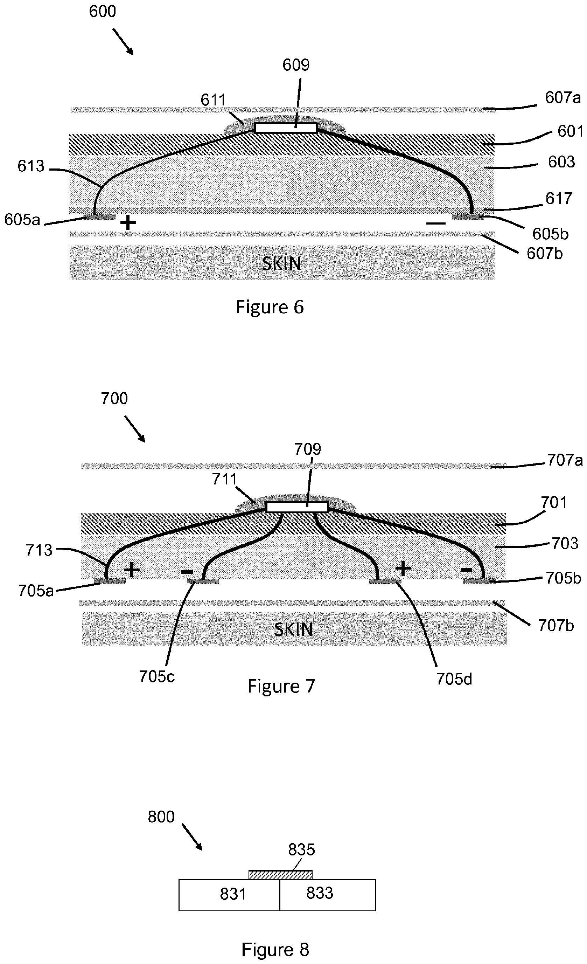

In some embodiments, the controller may control the flow of electrical current through the user's skin in order to cause one or more of (i) stimulation of nerves in the user's skin, (ii) stimulation of muscles proximal to the user's skin, (iii) an alteration of properties of the user's skin and (iv) an alteration of properties of blood vessels proximal to the user's skin. The flow of electrical current may provide pain relief, promote wound healing and/or promote absorption of a medicament by the user's skin. (i) may relate in particular to transcutaneous electrical nerve stimulation (TENS), and (ii) may relate to neuromuscular electrical stimulation (NMES). (iii) and (iv) are discussed below in relation to medicament absorption and Figure 6.

In some embodiments, the electrical stimulation driving electronics may be TENS driving electronics and the apparatus may be configured to enable a flow of electrical current through the user's skin at a level (e.g. an intensity, a frequency or a pattern) which is consistent with TENS therapy.

In other embodiments, the electrical stimulation driving electronics may be NMES driving electronics and the apparatus may be configured to enable a flow of electrical current through the user's skin at a level (e.g. an intensity, a frequency or a pattern) which is consistent with NMES.

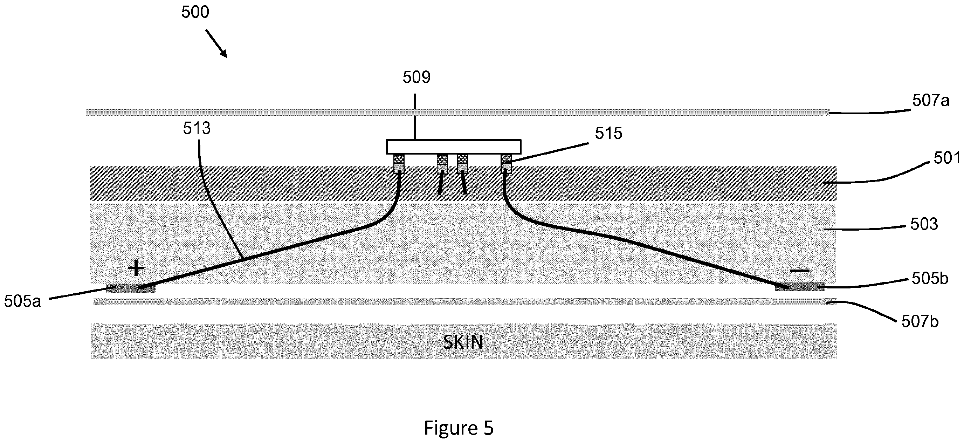

In some embodiments, the controller element 409 is fixedly attached to the rest of the apparatus (i.e. is not detachable/removable from the apparatus). In Figure 4, glob top encapsulation 411 (e.g. a layer of organic resin) is used to fixedly attach the controller element 409 to the rest of the apparatus but many other methods are known and are encompassed by the present disclosure. In other embodiments, such as the embodiment illustrated by Figure 5, the controlled element may be detachable from the rest of the apparatus and reused with another apparatus.

The porous substrate 403 may provide structural support for the proton battery 401 and the first pair of electrical contacts 405a, 405b. The substrate must be porous to allow for the passage of water from the user's skin through the porous substrate 403 to the proton battery 401. The porous substrate 403 (and also the proton battery 401) is preferably flexible to allow the apparatus 400 to be comfortably placed on a user's skin without, for example, pulling on the user's skin. Suitable porous substrate materials include woven textiles, Gore-Tex™ membranes and semi-permeable polymeric membranes.

The substrate may also prevent the proton battery 401 from being in direct contact with the user's skin when the apparatus 400 is attached to the user's skin. This may be preferable in order to avoid potential allergenic problems or other skin reactions to the materials in the proton battery 401.

To prevent the proton battery from being undesirably exposed to water prior to attachment to a user's skin (i.e. to prevent 'wastage' of power before the apparatus is intended to be used), the apparatus 400 may include one or more protective layers 407a, 407b made from an impermeable material and forming an impermeable barrier. In Figure 4, lower protective layer 407b is located on the bottom of the apparatus (i.e. the side which is intended to be placed in contact with the skin). To use the apparatus 400, a user would remove protective later 407b before attaching the apparatus 400 to the skin in order to allow water from the skin to reach the proton battery 401. Figure 4 also shows upper protective layer 407a on top of the apparatus (over the proton battery 401 and controller element 409) which prevents the proton battery 401 from being undesirably exposed to water from this side. In some embodiments, this upper protective layer 407a may be removed when the apparatus is attached to the skin to allow water from the environment (e.g. from the air) to reach the proton battery 401 in addition to water from the user's skin. In other embodiments, upper protective layer 407a may not be removable from the apparatus.

When suitably packaged (e.g. with two protective layers and further outer packaging), it may be possible to store the apparatus 400 for a long period of time (e.g. over 25 years) without significant degradation or loss of performance of the apparatus.

The apparatus 400 may be one or more of a patch (e.g. similar to a nicotine patch), a plaster, a sticker, a bandage (e.g. to be used around arms/legs or for whole body coverage), an elastic band or clothing such as socks. The apparatus may include attachment means such as an adhesive (e.g. similar to the adhesive on a nicotine patch) or glue to allow the apparatus to be temporarily attached to the user's skin whilst electrical stimulation is being provided. Various sizes of patches could be envisaged to allow provision of electrical stimulation to differently sized areas of a user's skin or to provide pain relief for different ailments.

The apparatus 400 may be disposable, i.e. not intended for repeated use. The useful lifetime of the apparatus may be the same as the effective lifetime of the proton battery (i.e. the time for which the proton battery can actively provide a sufficient/effective power output). In some embodiments, the apparatus may have a lifetime within the range of 10-60 minutes.

Alternatively, the apparatus may be reusable. For example, the apparatus may be sealed to prevent further exposure to water, thereby to prevent further active material consumption and power delivery, by replacing the removed protective layers and/or by placing the apparatus in a dry impermeable bag (e.g. a plastic bag).

The use of a single apparatus 400 including a power supply (proton battery), electrical contacts to a user's skin and connections therebetween provide several advantages over traditional TENS (or NMES) machines. The apparatus is simple to use and to apply, without the separate unit/wires/electrodes or remote controller parts of traditional machines. The apparatus is simple for a single user to apply to themselves without assistance. The user does not need to activate or control the operation of the machine (e.g. using a TENS unit or remote control) as the apparatus is automatically activated when it is placed in contact with the user's skin (and the moisture thereof). The apparatus may be small, discrete and comfortable to wear on the skin (e.g. as a patch or plaster). The apparatus may be more easily used in public, providing greater flexibility for a user as they are able to alleviate chronic and/or acute pain whilst outside the home or other private location.

Figure 5 shows another example of the present apparatus 500 including a proton battery 501, electrical contacts 505a and 505b, substrate 503, upper and lower protective layers 507a and 507b, controller element 509, connection wires 513 and magnetic contacts 515.

In some embodiments, the controller element 509 may be attachable to and detachable from the rest of the apparatus, rather than being completely integrated within the apparatus as in Figures 4, 6 and 7. The controller element 509 may be reusable and may be attached to and used to control/regulate the operation of multiple apparatuses in turn. This may be more cost efficient than providing each apparatus with a separate controller element.

For example, one reusable controller element 509 may be provided with a pack of 20 disposable patches. For each of the 20 patches, the proton battery within the disposable patch may provide power to the reusable controller element 509 which controls/regulates the flow of electrical current through the user's skin via the electrical contacts within the patch. Once the proton battery in a particular patch can no longer provide an effective power output (or once sufficient electrical stimulation has been provided), the reusable controller element 509 can be removed from that patch and is ready to be used with a new disposable patch (i.e. with a new proton battery).

Figure 5 shows one example of attaching the detachable controller element 509 to the rest of the apparatus 500 using respective magnetic contacts 515 within the controller element 509 and rest of the apparatus 500. Other attachment mechanisms (e.g. push-pull mechanical contact buttons) are possible and within the scope of the present disclosure.

Figure 6 shows another example of the present apparatus 600 including a proton battery 601, electrical contacts 605a and 605b, substrate 603, upper and lower protective layers 607a and 607b, controller element 609, glob top encapsulation 611, connection wires 613 and medicament 617.

Electrical stimulation of a user's skin may also be used to increase absorption of a medicament into the user's skin by changing properties of the user's skin and/or proximal blood vessels (e.g. by dilating blood vessels). In some embodiments, the apparatus may include a medicament layer 617 as shown in Figure 6. In Figure 6, medicament layer 617 is positioned above the electrical contacts and below the porous substrate 603 in order to be in close contact with the skin to facilitate absorption, though other positions are also possible. The medicament 617 may be comprised in or supported by a substrate layer which may be substrate 603 or a separate substrate layer.

Improved absorption of a medicament could alternatively be achieved using a separate medicament (e.g. a cream or gel) in conjunction with the present apparatus (with or without an integrated medicament layer.)

Figure 7 shows another example of the present apparatus 700 including a proton battery 701, first pair of electrical contacts 705a and 705b, substrate 703, upper and lower protective layers 707a and 707b, controller element 709, glob top encapsulation 711, connection wires 713 and second pair of electrical contacts 705c and 705d.

As shown in this example, the apparatus may comprise further pairs of electrical contacts (e.g. electrical contacts 705c, 705d) as well as the first pair of electrical contacts 705a, 705b. This may provide a better distribution of electrical stimulation (e.g. more homogenous coverage) across the area of the user's skin which is covered by the apparatus 700. This may be particularly advantageous for large apparatus sizes.

Figure 8 shows a cross-sectional view of one example of a proton battery 800. The energy generation mechanism of the proton battery 800 involves the degradation of graphene oxide when in contact with water from the user's skin. The proton battery 800 is a graphene oxide-based proton battery and comprises a first electrode 831 formed from graphene oxide, a second electrode 833 and an electrolyte 835. The first electrode 831 is configured to generate protons in the presence of water to produce a potential difference between the first 831 and second 833 electrodes. The electrolyte 835 is configured to enable the generated protons to flow from the first electrode 831 to the second electrode 833 when the first and second electrodes are connected by an external circuit. The potential difference produced by the generation of protons provides power to the electrical contacts 405a, 405b and the controller element 409, which may be considered to be the external circuit.

The proton battery 800 could include other elements such as a substrate configured to support the electrodes 831 and 833. The substrate may be a paper substrate, and the electrolyte may be soaked into the paper substrate. The use of a paper substrate 208 can reduce the fabrication costs and enable thinner (e.g. "two-dimensional") devices 201 to be produced. Polymers may also be used to form the supporting substrate 208.

In some embodiments, the second electrode may be formed from one or more of graphene oxide, reduced graphene oxide, potassium hydroxide, poly(3,4-ethylenedioxythiophene) polystyrene sulfonate, a base, and a conducting polymer.

In some embodiments, the electrolyte 835 may be configured to absorb water from the surrounding environment (e.g. from the user's skin or the air) and deliver it to the first electrode 331 to facilitate the generation of protons. This can be achieved by using electrolytes which are hydrophilic as well as ionically conductive. Such electrolytes include room-temperature ionic liquids and ion gels, examples of which include triethylsulfonium bis(trifluoromethylsulfonyl)imide, 1-buthyl-3-methyl-imidazolium, and trioctylmethylammonium bis(trifluoromethylsulfonyl)imide. The electrolyte may further comprise one or more salts configured to aid the flow of protons from the first electrode to the second electrode and/or enhance the absorption of water by the room-temperature ionic fluid from the surrounding environment. The one or more salts may comprise at least one of lithium bis(trifluoromethylsulfonyl)imide, lithium chloride and sodium chloride.

Multiple proton batteries (e.g. proton battery 800) may be connected in series to provide a larger total potential difference across the electrical contacts and thus a larger electrical current through the user's skin. Different potential power outputs can be achieved for different embodiments of the apparatus by using different types/numbers/combinations of proton batteries. In some embodiments, the total potential difference may be in the range of 0.1-10 V.

Of course, other types of proton batteries (and other types of graphene-oxide based batteries) are within the scope of the present disclosure.

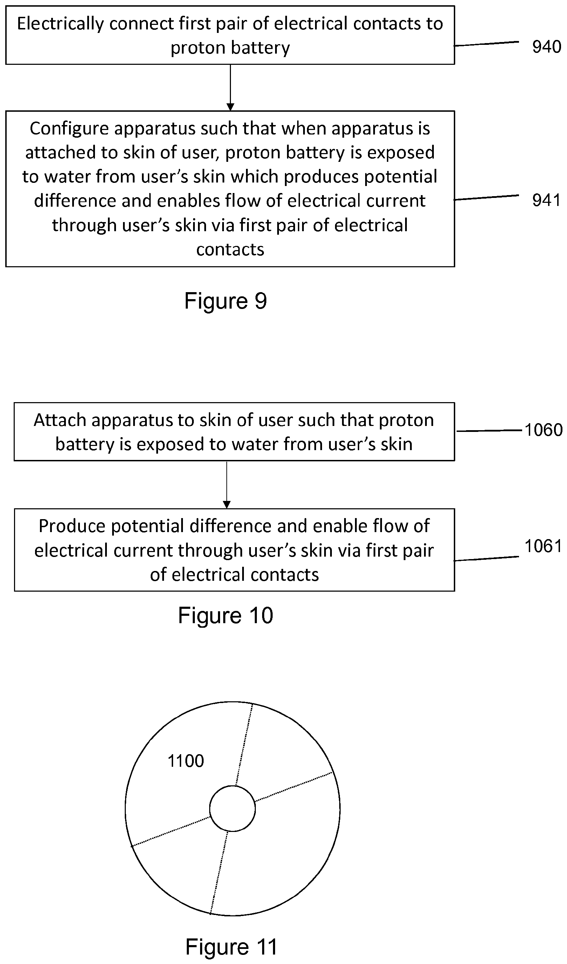

Figure 9 shows the main steps 940-941 of a method of making an apparatus described herein. The method generally comprises: electrically connecting a first pair of electrical contacts to a proton battery to enable a flow of electrical current therebetween when a potential difference from the proton battery is applied across the first pair of electrical contacts, the proton battery configured to generate protons in the presence of water to produce the potential difference 940; and configuring the apparatus such that when the apparatus is attached to the skin of a user, the proton battery is exposed to water from the user's skin which produces the potential difference and enables a flow of electrical current through the user's skin via the first pair of electrical contacts 941. The electrical contacts and proton battery materials can be directly placed onto a substrate (e.g. a textile, a Gore-Tex™ membrane or a semi-permeable polymeric membrane) by using ink-jet based technology, screen printing or a roll-to-roll process. These processes can simplify and reduce the cost of basic component manufacturing. The driving electronics are made separately and can be attached/laminated (e.g. glob-top encapsulated) at the end of the manufacturing chain.

Figure 10 shows the main steps 1060-1061 of a method of using an apparatus described herein. The method generally comprises: attaching the apparatus to the skin of a user such that the proton battery is exposed to water from the user's skin 1060; and producing a potential difference and enabling a flow of electrical current through the user's skin via the first pair of electrical contacts 1061.

Figure 11 illustrates schematically a computer/processor readable medium 1100 providing a program configured to perform, control or enable one or more of the method steps 940-941, 1060-1061 of Figure 9 or 10. In this example, the computer/processor readable medium is a disc such as a digital versatile disc (DVD) or a compact disc (CD). In some examples, the computer readable medium may be any medium that has been programmed in such a way as to carry out an inventive function. The computer program code may be distributed between the multiple memories of the same type, or multiple memories of a different type, such as ROM, RAM, flash, hard disk, solid state, etc.

Other embodiments depicted in the figures have been provided with reference numerals that correspond to similar features of earlier described embodiments. For example, feature number 101 can also correspond to numbers 201, 301 etc. These numbered features may appear in the figures but may not have been directly referred to within the description of these particular embodiments. These have still been provided in the figures to aid understanding of the further embodiments, particularly in relation to the features of similar earlier described embodiments.

It will be appreciated to the skilled reader that any mentioned apparatus/device and/or other features of particular mentioned apparatus/device may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/functional units.

In some embodiments, a particular mentioned apparatus/device may be pre-programmed with the appropriate software to carry out desired operations, and wherein the appropriate software can be enabled for use by a user downloading a "key", for example, to unlock/enable the software and its associated functionality. Advantages associated with such embodiments can include a reduced requirement to download data when further functionality is required for a device, and this can be useful in examples where a device is perceived to have sufficient capacity to store such pre-programmed software for functionality that may not be enabled by a user.

It will be appreciated that any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

It will be appreciated that any "computer" described herein can comprise a collection of one or more individual processors/processing elements that may or may not be located on the same circuit board, or the same region/position of a circuit board or even the same device. In some embodiments one or more of any mentioned processors may be distributed over a plurality of devices. The same or different processor/processing elements may perform one or more functions described herein.

It will be appreciated that the term "signalling" may refer to one or more signals transmitted as a series of transmitted and/or received signals. The series of signals may comprise one, two, three, four or even more individual signal components or distinct signals to make up said signalling. Some or all of these individual signals may be transmitted/received simultaneously, in sequence, and/or such that they temporally overlap one another.

With reference to any discussion of any mentioned computer and/or processor and memory (e.g. including ROM, CD-ROM etc), these may comprise a computer processor, Application Specific Integrated Circuit (ASIC), field-programmable gate array (FPGA), and/or other hardware components that have been programmed in such a way to carry out the inventive function.

## Claims

1. An apparatus comprising:
a proton battery configured to generate protons in the presence of water to produce a potential difference; and
a first pair of electrical contacts electrically connected to the proton battery to enable a flow of electrical current therebetween when the potential difference from the proton battery is applied across the first pair of electrical contacts,
wherein the apparatus is configured such that when the apparatus is attached to the skin of a user, the proton battery is exposed to water from the user's skin which produces the potential difference and enables a flow of electrical current through the user's skin via the first pair of electrical contacts.

2. The apparatus of claim 1, wherein the proton battery is a graphene thin film battery.

3. The apparatus of claim 1, wherein the proton battery comprises a first electrode, a second electrode and an electrolyte,
the first electrode comprising graphene oxide and configured to generate protons in the presence of water to produce the potential difference between the first and second electrodes, and
the electrolyte configured to enable the generated protons to flow from the first electrode to the second electrode when the first and second electrodes are connected by an external circuit.

4. The apparatus of claim 3, wherein the electrolyte is configured to absorb water from the surrounding environment and deliver it to the first electrode to facilitate the generation of protons.

5. The apparatus of claim 1, further comprising a porous substrate and wherein the apparatus is configured such that when the apparatus is attached to the skin of a user, the proton battery is exposed to water from the user's skin via the porous substrate.

6. The apparatus of claim 1, further comprising a controller element which is electrically connected between the proton battery and the first pair of electrical contacts and is configured to control the potential difference applied across the first pair of electrical contacts by the proton battery and the resulting flow of electrical current through the user's skin.

7. The apparatus of claim 6, wherein the controller element comprises electrical stimulation driving electronics which control the flow of electrical current through the user's skin according to a predetermined pattern.

8. The apparatus of claim 6, wherein the apparatus is configured to enable a flow of electrical current through the user's skin at a level which causes one or more of:
stimulation of nerves in the user's skin;
stimulation of muscles proximal to the user's skin;
alteration of properties of the user's skin; and
alteration of properties of blood vessels proximal to the user's skin.

9. The apparatus of claim 7, wherein the electrical stimulation driving electronics are transcutaneous electrical nerve stimulation driving electronics, and wherein the apparatus is configured to enable a flow of electrical current through the user's skin at a level which is consistent with transcutaneous electrical nerve stimulation therapy.

10. The apparatus of claim 6, wherein the controller element is one of detachable from the apparatus and not detachable from the apparatus.

11. The apparatus of claim 10, wherein the controller element is detachable from the apparatus via respective magnetic contacts within the controller element and the apparatus.

12. The apparatus of claim 1, further comprising one or more additional pairs of electrical contacts electrically connected to the proton battery to enable a flow of electrical current through the user's skin via the respective one or more pairs of electrical contacts when the potential difference from the battery is applied across the respective one or more additional pairs of electrical contacts.

13. The apparatus of claim 1, further comprising a medicament configured to be absorbed into the user's skin, wherein the apparatus is configured such that the flow of electrical current through the user's skin improves the absorption of the medicament into the user's skin.

14. The apparatus of claim 1, wherein the apparatus comprises one or more of a patch, a plaster, a sticker, a bandage, an elastic band, clothing and socks.

15. A method of making an apparatus, the method comprising:
electrically connecting a first pair of electrical contacts to a proton battery to enable a flow of electrical current therebetween when a potential difference from the proton battery is applied across the first pair of electrical contacts, the proton battery configured to generate protons in the presence of water to produce the potential difference; and
configuring the apparatus such that when the apparatus is attached to the skin of a user, the proton battery is exposed to water from the user's skin which produces the potential difference and enables a flow of electrical current through the user's skin via the first pair of electrical contacts.

## Patentansprüche

1. Vorrichtung, umfassend:
eine Protonenbatterie, die zum Erzeugen von Protonen in Anwesenheit von Wasser zur Erzeugung einer Potentialdifferenz ausgelegt ist; und
ein erstes Paar elektrischer Kontakte, die elektrisch mit der Protonenbatterie verbunden sind, um einen elektrischen Stromfluss zwischen ihnen zu ermöglichen, wenn die Potentialdifferenz von der Protonenbatterie über das erste Paar elektrischer Kontakte angelegt wird,
wobei die Vorrichtung derart ausgelegt ist, dass, wenn die Vorrichtung an der Haut eines Benutzers befestigt ist, die Protonenbatterie Wasser aus der Haut des Benutzers ausgesetzt wird, wodurch die Potentialdifferenz erzeugt wird und ein elektrischer Stromfluss durch die Haut des Benutzers über das erste Paar elektrischer Kontakte ermöglicht wird.

2. Vorrichtung nach Anspruch 1, wobei die Protonenbatterie eine Graphen-Dünnfilm-Batterie ist.

3. Vorrichtung nach Anspruch 1, wobei die Protonenbatterie eine erste Elektrode, eine zweite Elektrode und einen Elektrolyten umfasst,
wobei die erste Elektrode Graphenoxid umfasst und zur Erzeugung von Protonen im Anwesenheit von Wasser ausgelegt ist, um die Potentialdifferenz zwischen den ersten und zweiten Elektroden zu erzeugen, und
wobei der Elektrolyt dazu ausgelegt ist, das Strömen der erzeugten Protonen von der ersten Elektrode zur zweiten Elektrode zu ermöglichen, wenn die ersten und zweiten Elektroden über einen externen Schaltkreis miteinander verbunden sind.

4. Vorrichtung nach Anspruch 3, wobei der Elektrolyt dazu ausgelegt ist, Wasser aus dem umgebenden Milieu zu absorbieren und an die erste Elektrode abzugeben, um die Erzeugung von Protonen zu erleichtern.

5. Vorrichtung nach Anspruch 1, ferner umfassend ein poröses Substrat und wobei die Vorrichtung derart ausgelegt ist, dass, wenn die Vorrichtung an der Haut eines Benutzers befestigt ist, die Protonenbatterie Wasser aus der Haut des Benutzers über das poröse Substrat ausgesetzt wird.

6. Vorrichtung nach Anspruch 1, ferner umfassend ein Steuerelement, das zwischen der Protonenbatterie und dem ersten Paar elektrischer Kontakte angeschlossen ist und dazu ausgelegt ist, die über das erste Paar elektrischer Kontakte von der Protonenbatterie angelegte Potentialdifferenz und den resultierenden elektrischen Stromfluss durch die Haut des Benutzers zu steuern.

7. Vorrichtung nach Anspruch 6, wobei das Steuerelement elektrische Stimulationsantriebselektronik umfasst, die den elektrischen Stromfluss durch Haut des Benutzers nach einem vorbestimmten Muster steuert.

8. Vorrichtung nach Anspruch 6, wobei die Vorrichtung dazu ausgelegt ist, einen elektrischen Stromfluss durch die Haut des Benutzers auf einem Niveau zu ermöglichen, das eines oder mehrere der Folgenden verursacht:
Stimulation von Nerven in der Haut des Benutzers;
Stimulation von Muskeln proximal zur Haut des Benutzers;
Veränderung von Eigenschaften der Haut des Benutzers, und
Veränderung von Eigenschaften von Blutgefäßen neben der Haut des Benutzers.

9. Vorrichtung nach Anspruch 7, wobei die elektrische Stimulationsantriebselektronik transkutane elektrische Nervenstimulationsantriebselektronik ist und wobei die Vorrichtung dazu ausgelegt ist, einen elektrischen Stromfluss durch die Haut des Benutzers auf einem Niveau zu ermöglichen, das mit transkutaner elektrischer Nervenstimulationstherapie in Einklang ist.

10. Vorrichtung nach Anspruch 6, wobei das Steuerelement entweder von der Vorrichtung abnehmbar ist oder nicht von der Vorrichtung abnehmbar ist.

11. Vorrichtung nach Anspruch 10, wobei das Steuerelement über jeweilige magnetische Kontakte innerhalb des Steuerelements und der Vorrichtung von der Vorrichtung abnehmbar ist.

12. Vorrichtung nach Anspruch 1, ferner umfassend ein oder mehr zusätzliche Paare elektrischer Kontakte, die elektrisch mit der Protonenbatterie verbunden sind, um einen elektrischen Stromfluss durch die Haut des Benutzers über die jeweiligen ein oder mehr Paare elektrischer Kontakte zu ermöglichen, wenn die Potentialdifferenz von der Protonenbatterie über die jeweiligen ein oder mehr zusätzlichen Paare elektrischer Kontakte angelegt wird.

13. Vorrichtung nach Anspruch 1, ferner umfassend ein Medikament, das dazu ausgelegt ist, in die Haut des Benutzers absorbiert zu werden, wobei die Vorrichtung derart ausgelegt ist, dass der elektrische Stromfluss durch die Haut des Benutzers die Absorption des Medikaments in die Haut des Benutzers verbessert.

14. Vorrichtung nach Anspruch 1, wobei die Vorrichtung ein oder mehrere eines Patches, eines Pflasters, eines Stickers, eines Verbands, eines elastischen Bands, von Bekleidung und von Socken umfasst.

15. Verfahren zur Herstellung einer Vorrichtung, wobei das Verfahren das Folgende umfasst:
elektrisches Verbinden eines ersten Paars elektrischer Kontakte mit einer Protonenbatterie, um einen elektrischen Stromfluss zwischen ihnen zu ermöglichen, wenn die Potentialdifferenz von der Protonenbatterie über das erste Paar elektrischer Kontakte angelegt wird, wobei die Protonenbatterie zum Erzeugen von Protonen in Anwesenheit von Wasser zur Erzeugung der Potentialdifferenz ausgelegt ist; und
Konfigurieren der Vorrichtung auf eine solche Art, dass, wenn die Vorrichtung an der Haut eines Benutzers befestigt ist, die Protonenbatterie Wasser aus der Haut des Benutzers ausgesetzt wird, wodurch die Potentialdifferenz erzeugt wird und ein elektrischer Stromfluss durch die Haut des Benutzers über das erste Paar elektrischer Kontakte ermöglicht wird.

## Revendications

1. Appareil, comprenant :
une batterie à protons configurée pour générer des protons en présence d'eau pour produire une différence de potentiel ; et
une première paire de contacts électriques électriquement connectés à la batterie à protons pour permettre un passage de courant électrique entre ceux-ci lorsque la différence de potentiel provenant de la batterie à protons est appliquée sur la première paire de contacts électriques,
dans lequel l'appareil est configuré de telle sorte que, lorsque l'appareil est attaché à la peau d'un utilisateur, la batterie à protons soit exposée à de l'eau, provenant de la peau de l'utilisateur, qui produit la différence de potentiel et permet un passage de courant électrique à travers la peau de l'utilisateur par l'intermédiaire de la première paire de contacts électriques.

2. Appareil selon la revendication 1, dans lequel la batterie à protons est une batterie à film mince de graphène.

3. Appareil selon la revendication 1, dans lequel la batterie à protons comprend une première électrode, une seconde électrode et un électrolyte,
la première électrode comprenant de l'oxyde de graphène et étant configurée pour générer des protons en présence d'eau pour produire la différence de potentiel entre les première et seconde électrodes, et
l'électrolyte étant configuré pour permettre aux protons générés de passer de la première électrode à la seconde électrode lorsque les première et seconde électrodes sont connectées par un circuit externe.

4. Appareil selon la revendication 3, dans lequel l'électrolyte est configuré pour absorber de l'eau de l'environnement et la fournir à la première électrode pour faciliter la génération de protons.

5. Appareil selon la revendication 1, comprenant en outre un substrat poreux et dans lequel l'appareil est configuré de telle sorte que, lorsque l'appareil est attaché à la peau d'un utilisateur, la batterie à protons soit exposée à de l'eau de la peau de l'utilisateur par l'intermédiaire du substrat poreux.

6. Appareil selon la revendication 1, comprenant en outre un élément de commande qui est électriquement connecté entre la batterie à protons et la première paire de contacts électriques et est configuré pour commander la différence de potentiel appliquée sur la première paire de contacts électriques par la batterie à protons et le passage résultant de courant électrique à travers la peau de l'utilisateur.

7. Appareil selon la revendication 6, dans lequel l'élément de commande comprend des composants électroniques d'excitation de stimulation électrique qui commandent le passage de courant électrique à travers la peau de l'utilisateur selon un motif prédéterminé.

8. Appareil selon la revendication 6, dans lequel l'appareil est configurée pour permettre un passage de courant électrique à travers la peau de l'utilisateur à un niveau qui cause une ou plusieurs de :
stimulation de nerfs dans la peau de l'utilisateur ;
stimulation de muscles à proximité de la peau de l'utilisateur ;
altération de propriétés de la peau de l'utilisateur ; et
altération de propriétés de vaisseaux sanguins à proximité de la peau de l'utilisateur.

9. Appareil selon la revendication 7, dans lequel les composants électroniques d'excitation de stimulation électrique sont des composants électroniques d'excitation de stimulation de nerf électriques transcutanés, et dans lequel l'appareil est configuré pour permettre un passage de courant électrique à travers la peau de l'utilisateur à un niveau qui est cohérent avec une thérapie de stimulation de nerf électrique transcutanée.

10. Appareil selon la revendication 6, dans lequel l'élément de commande est un de détachable de l'appareil et de non détachable de l'appareil.

11. Appareil selon la revendication 10, dans lequel l'élément de commande est détachable de l'appareil par l'intermédiaire de contacts magnétiques respectifs à l'intérieur de l'élément de commande et l'appareil.

12. Appareil selon la revendication 1, comprenant en outre une ou plusieurs paires supplémentaires de contacts électriques électriquement connectés à la batterie à protons pour permettre un passage de courant électrique à travers la peau de l'utilisateur par l'intermédiaire des une ou plusieurs paires respectives de contacts électriques lorsque la différence de potentiel provenant de la batterie est appliquée sur les une ou plusieurs paires supplémentaires respectives de contacts électriques.

13. Appareil selon la revendication 1, comprenant en outre un médicament configuré pour être absorbé dans la peau de l'utilisateur, dans lequel l'appareil est configuré de telle sorte que le passage de courant électrique à travers la peau de l'utilisateur améliore l'absorption du médicament dans la peau de l'utilisateur.

14. Appareil selon la revendication 1, dans lequel l'appareil comprend un ou plusieurs d'une pièce, d'un pansement, d'un autocollant, d'un bandage, d'une bande élastique, de vêtements et de chaussettes.

15. Procédé de fabrication d'un appareil, le procédé comprenant :
la connexion électrique d'une première paire de contacts électriques à une batterie à protons pour permettre un passage de courant électrique entre ceux-ci lorsqu'une différence de potentiel provenant de la batterie à protons est appliquée sur la première paire de contacts électriques, la batterie à protons étant configurée pour générer des protons en présence d'eau pour produire la différence de potentiel ; et
la configuration de l'appareil de telle sorte que, lorsque l'appareil est attaché à la peau d'un utilisateur, la batterie à protons soit exposée à de l'eau de la peau de l'utilisateur qui produit la différence de potentiel et permet un passage de courant électrique à travers la peau de l'utilisateur par l'intermédiaire de la première paire de contacts électriques.
